# EUROPÄISCHE PATENTANMELDUNG

(11) **EP 1 203 576 A1**
(43) Veröffentlichungstag der Anmeldung: **08.05.2002**
(21) Anmeldenummer: 01125501.5
(22) Anmeldetag: 25.10.2001
(51) Int. Cl.: A61K 7/30

(54) **Reinigungsmittel für Zahnprothesen**

(30) Priorität: 03.11.2000 DE 10054693
(71) Anmelder: Clariant GmbH, 65929 Frankfurt am Main (DE)
(72) Erfinder: Reinhardt, Gerd, Dr., 65779 Kelkheim (DE); Henning, Torsten, Dr., 65812 Bad Soden (DE); Johannpeter, Wiebke, 61449 Steinbach (DE)
(74) Vertreter: Otto, Adalbert, Dr.

(57) **Zusammenfassung**

Reinigungsmittel für Zahnprothesen enthaltend ein Oxidationsmittel und eine organische Verbindung, die eine Carbonylgruppe trägt welche in wässriger Lösung durch Reaktion mit dem Oxidationsmittel eine Dioxiran-Struktur ausbildet.

## Beschreibung

Die Erfindung betrifft ein pulver- oder tablettenförmiges Reinigungsmittel zum selbsttätigen Reinigen und Desinfizieren von Zahnprothesen in wässeriger Lösung.

Reinigungsmittel für Zahnprothesen werden in der Weise verwendet, dass sie zugleich mit der zu reinigenden Zahnprothese in Wasser eingelegt werden, woraufhin die Reinigungsmittel dann unter mehr oder weniger starker Gasentwicklung zerfallen und Wirkstoffe freigeben, welche die auf der Zahnprothese anhaftenden Schmutzbeläge angreifen und entfernen.

Derartige Reinigungsmittel sind z.B. beschrieben in DE-23 12 847, 25 48 469, 26 58 450 und 27 41 072, in EP-00 10 412, 00 28 005 und 0102 418 sowie in EP-01 33 354, 0149 308, 0157 464 und 0225 658. Sie enthalten im wesentlichen sauerstoffabgebende Substanzen zum Bleichen, Oxidieren und Desinfizieren, anorganische Enthärter und Kalkbinder, Chelatbildner, gasbildende Träger, wie Carbonate und/oder Bicarbonate, Zitronensäure, Weinsäure, Amidosulfonsäure bzw. deren Salze, Desinfektionsmittel, wie Benzoate und/oder Hydroxybenzoesäurealkylester, sowie Gleit-, Binde- und/oder Sprengmittel, Tenside, Farbstoffzusätze und schließlich Aromamasse.

Die pH-Werte bei Auflösung in Wasser schwanken, je nach Zusammensetzung, zwischen sauer, neutral und alkalisch. In den meisten Fällen handelt es sich um Schnellreinigungsmittel, d. h. solche mit einer Reinigungszeit von 10 bis 15 Minuten; in einigen Fällen handelt es sich aber auch um langsam wirkende Produkte mit relativ langen Anwendungszeiten, bei denen die volle Reinigungswirkung hinsichtlich der zu behandelnden Zahnprothese erst in etwa 1 bis 2 Stunden erreicht wird.

Ein Nachteil dieser Reinigungsmittel ist ihre geringe reinigende und bleichende Wirkung im Temperaturbereich unterhalb 60°C. In EP-02 53 772 ist daher eine Reinigungstablette beschrieben, die zusätzlich einen Bleichaktivator wie Tetraacetylethylendiamin (TAED) in Konzentrationen von 5 bis 15 Gew.-% enthält. Durch den bei der Reinigungstablette eingesetzten Bleichaktivator wird in wässriger Lösung ein Reaktionsmechanismus initiiert, wobei durch Reaktion des Aktivators mit Perhydroxylanionen als Zwischenstufe Peressigsäure gebildet wird, die stark bleichende und desinfizierende Eigenschaften aufweist.

Überraschenderweise wurde nun gefunden, dass sich die Wirksamkeit von Reinigungsmitteln für Zahnprothesen dadurch deutlich steigern lässt, dass man eine Carbonylfunktion enthaltende organische Substanz hinzufügt, die in der Lage ist, mit dem vorhandenen Oxidationsmittel intermediär eine Dioxiran-Struktur auszubilden.

Gegenstand der Erfindung sind deshalb Reinigungsmittel für Zahnprothesen, enthaltend
a) mindestens ein organisches oder anorganischen Oxidationsmittel und
b) mindestens eine carbonylgruppenhaltige organische Substanz, die in wässriger Lösung durch Reaktion mit dem Oxidationsmittel eine Dioxiran-Struktur bildet.

Unter einer Dioxiran-Struktur wird dabei ein Dreiring bestehend aus einem Kohlenstoffatom und zwei Sauerstoffatomen verstanden. Dioxirane können bekanntermaßen durch Umsetzung von Peroxomonosulfonsäuren oder deren Salze mit bestimmten Carbonylgruppen tragenden organischen Verbindungen erhalten werden. Dioxirane übertragen dann ein Sauerstoffatom auf den der Zahnprothese anhaftenden Schmutz. Nach erfolgtem Bleichprozess bildet sich die Carbonylverbindung zurück und steht für einen neuen Cyclus zur Verfügung. Von Vorteil hierbei ist, dass die Cabonylkomponente nur in geringen Konzentrationen eingesetzt werden muss, da sie, zumindest im Idealfall, katalytisch wirksam ist.

Die Verwendung von Dioxiranen in der präparativen organischen Chemie ist seit einigen Jahren bekannt. Beispiele hierzu sind in Bull. Soc. Chem. Belg., Vol 105, Heft 10-11, S.581-597 und in der dort zitierter Literatur beschrieben. Weiterhin ist bekannt, dass Mischungen aus Peroxymonosulfonsäuren und bestimmten Carbonylverbindungen zum Bleichen von Textilien und harten Oberflächen verwendet werden können. Beispiele hierzu sind in US 3,822,114 und WO99/23197 aufgezeigt. Aus keiner der genannten Publikationen ist jedoch ersichtlich, dass Dioxirane, hergestellt durch Umsetzung von Peroxysulfonsäuren bzw. deren Salzen und bestimmten Carbonylverbindungen, exzellente Wirksamkeit beim Reinigen von Zahnprothesen aufweisen.

Gegenstand der Erfindung ist weiterhin ein Verfahren zum Bleichen und Desinfizieren von Zahnprothesen, dadurch gekennzeichnet, dass man die Zahnprothese mit einer wässrigen Lösung eines Reinigungsmittels behandelt, das mindestens ein organisches oder anorganisches Oxidationsmittel und mindestens eine carbonylgruppenhaltige organische Substanz, die in wässriger Lösung durch Reaktion mit dem Oxidationsmittel eine Dioxiran-Struktur bildet, enthält.

Als carbonylgruppenhaltige organische Substanzen kommen vorzugsweise Verbindungen der Formel 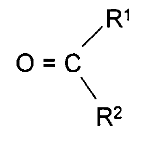 infrage, aus denen durch Reaktion mit dem Oxidationsmittel die Dioxiranverbindungen der Formel 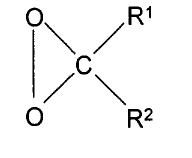 entstehen. R¹ und R² bedeuten C₁-C₂₄-Alkyl-, C₂-C₂₄-Alkenyl- oder Arylgruppen, die durch Halogenatome wie F, Cl, Br oder J, Nitro-, Cyano- oder Aminogruppen oder Gruppen der Formeln -N(R³)₂ oder 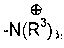 ,wobei R³ C₁-C₄-Alkyl, Benzyl oder Phenyl ist, substituiert sein können.

Die Reste R¹ und R² können auch Bestandteile ein und desselben Ringsystems sein. Besonders bevorzugt sind hierbei Ringsysteme, in denen R₁ und R₂ in Summe 4-7 Kohlenstoffatome aufweisen. Solche cyclischen Verbindungen, in denen R₁ und R₂ Bestandteil eines Ringsystems sind, können weiterhin substituiert sein. Als Verbindungen der Carbonylstruktur sind besonders bevorzugt: Dimethylketon, Methylethylketon, 1,1,1,-Trifluoraceton, Perfluoraceton, Diphenylketon, Alkylphenylketon, Dipyridylketon, Dipyridylketon-N-oxid. Aus der Gruppe der cyclischen Ketone werden bevorzugt: Cyclopentanon, Cyclohexanon, Cycloheptanon, Cyclooctanon und deren Methylderivate sowie heterocyclische Ketone wie z.B. N-Acetyl-4-piperidon.

Ebenfalls besonders bevorzugt sind aminsubstituierte Ketone der allgemeinen Formeln (III) und (IV) bzw. die analogen ammoniumsubstituierten Verbindungen der Formeln (V) und (VI) 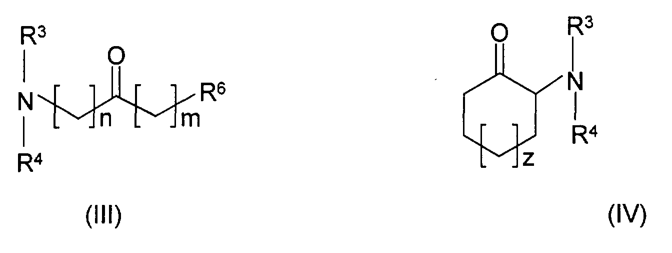 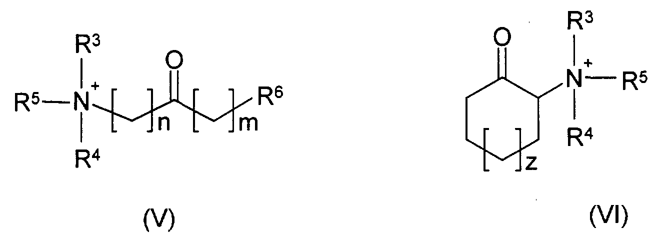 wobei R³, R⁴, R⁵ und R⁶ C₁-C₂₂-Alkyl, C₂-C₂₂-Alkenyl oder Aryl bedeutet. n = 1-11, bevorzugt 1-5, insbesondere 1-2, m = 0-12, bevorzugt 0-3, z = 0, 1,2.

Die positiv geladene Ammoniumketone können zum Ladungsausgleich negative geladene Gegenionen enthalten aus der Gruppe der Halogenide wie Cl⁻, Br⁻ oder ROSO⁻₃, TosO⁻, SO₄²⁻ oder CO₃²⁻, wobei R C₁-C₄-Alkyl und Tos Tosylat bedeutet.

Beispiele für derartige Verbindungen der Formeln III bis VI sind N,N-Dimethylaminoaceton, N,N-Diethylaminoaceton, N,N-Dipropylaminoaceton, N,N-Dibutylaminoaceton, N,N-Dihexylaminoaceton, N,N-Dioctylaminoaceton, N,N-Didecylaminoaceton, N,N-Didodecylaminoaceton, N,N-Sterylaminoaceton, N-Methyl-N-ethylaminoaceton, N-Methyl-N-propylaminoaceton und N-Methyl-N-butylaminoaceton, 2-(N,N-Dimethylamino)-cyclohexanon oder 4-(N,N-Dimethylamino)-cyclohexanon und deren höhere Homologe.

Die carbonylgruppenhaltigen Verbindungen werden in den erfindungsgemäßen Reinigungsmittel in Konzentrationen von 0,01 - 10 %, vorzugsweise 0,1 - 8 % und insbesondere 0,5 - 5 % eingesetzt.

Als anorganische Oxidationsmittel auf Sauerstoffbasis kommen in erster Linie alle Alkalimetall- oder Ammoniumperoxosulfate in Betracht, wie z.B. Kaliumperoxomonosulfat (technisch: Caroat® oder Oxone® ). Daneben können aber auch Alkaliperborat-mono beziehungsweise -tetrahydrate und/oder Alkalipercarbonate, wobei Natrium das bevorzugte Alkalimetall ist, verwendet werden. In einer besonders bevorzugten Ausführungsform werden Mischungen von Peroxosulfaten mit Perboraten oder Percarbonaten im Mischungsverhältnis 1:10 bis 10:1, bevorzugt 1:5 bis 5:1 verwendet. Zusätzlich oder alternativ können die erfindungsgemäßen Reinigungsmittel Oxidationsmittel auf organischer Basis im Konzentrationsbereich von 1 - 20 % enthalten. Hierzu zählen alle bekannte Peroxycarbonsäuren, z.B. Monoperoxyphthalsäure, Dodecandiperoxysäure oder Phthalimidoperoxycarbonsäuren (PAP). Die Konzentration der Oxidationsmittel in den erfindungsgemäßen Reinigungsmitteln beträgt 5 - 90 %, vorzugsweise 10 -70 %.

Zusätzlich zu den eingesetzten Oxidationsmitteln und carbonylgruppenhaltigen Verbindungen können die erfindungsgemäßen Reinigungsmittel einen oder mehrere Bleichaktivatoren enthalten. Dies sind Verbindungen, die unter Perhydrolysebedingungen Peroxycarbonsäuren freisetzen. Geeignet sind die üblichen Bleichaktivatoren, die O- und/oder N-Acylgruppen enthalten. Bevorzugt sind mehrfach acylierte Alkylendiamine, insbesondere Tetraacetylethylendiamin (TAED), acylierte Glykolurile, insbesondere Tetraacetylglykoluril (TAGU), acylierte Triazinderivate , insbesondere 1,5-Diacetyl-2,4-dioxohexahydro-1,3,5-triazin (DADHT), acylierte Phenolsulfonate, insbesondere Nonanoyl- oder Isononanoyloxybenzolsulfonat (NOBS bzw. ISONOBS), acylierte mehrwertige Alkohole, insbesondere Triacetin, Ethylenglykoldiacetat und 2,5-Diacetoxy-2,5-dihydrofuran sowie acetyliertes Sorbit und Mannit, und acylierte Zuckerderivate, insbesondere Pentaacetylglukose (PAG), Pentaacetylfructose, Tetraacetylxylose und Octaacetyllactose. Weitere mögliche Aktivatoren sind in WO 98/11880 genannt. Auch die aus DE-44 43 177 bekannten Kombinationen konventioneller Bleichaktivatoren können eingesetzt werden.

Eine weitere wichtige Gruppe von Bleichaktivatoren stellen Nitrilgruppen-haltige Verbindungen dar. Hierzu gehören Cyanamid und dessen Derivate wie N-Cyanopiperidin, N-Cyanopyrrolidin, N-Cyanomorpholin und Cyanopyridine. Eine besonders bevorzugte Klasse von Aktivatoren stellen offenkettige oder cyclische Nitrilquats dar, wie sie z.B. in EP-A-303 520, EP-A-464 880 und WO 98/23602 beschrieben sind. Besonders bevorzugt sind Trimethylammoniumacetonitril und Morpholinomethylammoniumacetonitril. Die verwendeten Aktivatoren können in Konzentrationen von 0,01 - 25 %, vorzugsweise 0,5 - 15 % eingesetzt werden.

Überdies kann der Zusatz geringer Mengen bekannter Bleichmittelstabilisatoren wie beispielsweise von Phosphonaten oder Aminocarboxylaten von Vorteil sein.

Neben den bereits genannten Komponenten enthalten die erfindungsgemäßen Reinigungsmittel die üblichen Bestandteile wie eingangs beschrieben. Als gasfreisetzendes System kann das Reinigungsmittel eine Kombination eines Alkalimetallcarbonates bzw. -bicarbonat mit einer anorganischen oder organischen Säure enthalten. Als Alkalimetallcarbonat bzw. -bicarbonat können verwendet werden Natriumcarbonat, Kaliumcarbonat, Natriumbicarbonat, Natriumsesquicarbonat, Kaliumbicarbonat oder Mischungen der genannten Stoffe. Als Säurekomponente können folgende Verbindungen zum Einsatz kommen: Sulfamidsäure, saure Salze der Phosphorsäure und der Schwefelsäure. Als organische Säure können verwendet werden Zitronensäure, Äpfelsäure, Maleinsäure oder Fumarsäure. Die Mischung aus Säure und Base führt bei Wasserzugabe zu Bildung von Gasbläschen, die die reinigende Wirkung der Oxidationsmittel unterstützen. Die Carbonat oder Bicarbonat-Komponente wird in Konzentrationen von 5 bis 70 %, vorzugsweise von 20 bis 60 % eingesetzt, die saure Komponente in Konzentrationen von 5 bis 50 %, vorzugsweise von 10 bis 40 %.

Weiterhin kann es sinnvoll sein, dass das Reinigungsmittel weitere Komponenten enthält, um die Reinigungswirkung zu verbessern. Hierzu gehören zum Beispiel oberflächenaktive Substanzen wie anionische, nichtionische oder kationische Tenside, Komplexbildner, Enzyme, Geschmacks- und Geruchsstoffe, Farbstoffe, Konservierungsmittel etc.

Typische Komponenten solcher Reinigungsmittel und deren Einsatzkonzentrationen sind in der folgenden Tabelle wiedergegeben:

| | |
|---|---|
| Natriumperboratmonohydrat | 15 - 30 Gew.-% |
| Kaliummonopersulfat | 20 - 40 Gew.-% |
| Natriumhydrogencarbonat | 10 - 30 Gew.-% |
| Natriumcarbonat | 0 - 10 Gew.-% |
| Natriumsulfat | 0 - 10 Gew.-% |
| Zitronensäure bzw. Citratsalze o.ä. | 5 -20 Gew.-% |
| Tetraacetylethylendiamin | 0,3 - 3 Gew.-% |
| organische Phosphonsäure bzw. deren Salze | 0,5 - 3 Gew.-% |
| Polyethylenglykol 20 000 | 1 - 4,5 Gew.-% |
| Polyvinylpyrrolidon | 0 - 3 Gew.-% |
| Siliciumdioxid (Aerosil 200/300) | 0,5 - 1,5 Gew.-% |
| Natriumdodecylbenzolsulfonat Ricinylmonoethanolamid- | |
| sulfosuccinat, Dinatrium Salz oder ähnlich wirkende Tenside | 0,2- 1,5 Gew.-% |
| Gehärtete Triglyceride | 0 - 2 Gew.-% |
| Fettalkoholpolyglykoläther | 0,5 - 2 Gew.-% |
| Konservierungsmittel, gleichzeitig Stabilisator | 0 - 2 Gew.-% |
| Pfefferminzpulver bzw. andere Aromastoffe (z. B. Eukalyptus) | 1 - 2 Gew.-% |
| Farbstoffsystem | 0,05-0,35 Gew.-% |

Nachstehend ist eine Reinigungstablette nach der Erfindung anhand eines konkreten Ausführungsbeispieles beschrieben.

| | |
|---|---|
| 30 Gew.-% | Natriumperboratmonohydrat |
| 20 Gew.-% | Kaliummonopersulfat |
| 20 Gew.-% | Natriumhydrogencarbonat |
| 5 Gew.-% | Natriumcarbonat |
| 4 Gew.-% | Natriumsulfat |
| 7 Gew.-% | Zitronensäure, Natriumsalz |
| 1,5 Gew.-% | Dipyridylketon |
| 1,5 Gew.-% | organische Phosphonsäuren bzw. deren Salze |
| 4 Gew.-% | Polyethylenglykol 20 000 |
| 1,5 Gew.-% | Polyvinylpyrrolidon |
| 1,5 Gew.-% | Aerosil® 200/300 |
| 0,75 Gew.-% | Natriumdodecylbenzolsulfonat |
| 0,5 Gew.-% | Gehärtete Triglyceride |
| 1 Gew.-% | Fettalkoholpolyglykoläther |
| 1 Gew.-% | Konservierungsmittel |
| 0,5 Gew.-% | Pfefferminzpulver, und |
| 0,25 Gew.-% | Indigotin® L-Blue 2 und Chinolingelb® L-Gelb 3, |

wurden als Pulver bzw. in granulierter Form mit bekannten Techniken zu einer Reinigungstablette verpresst. Die so entstandene Reinigungstablette zeigte nach dem Auflösen bei einer Temperatur der Reinigungsflotte von 30°C, einen pH-Wert in der Reinigungsflotte von 9,0, wobei der Reinigungsprozess, in seinem Ablauf voll optisch verfolgbar durch entsprechenden Farbumschlag, nach etwa 10 Minuten vollständig abgeschlossen war.

### Beispiele

### Bleichversuche

Die Verbesserung der Bleichleistung der erfindungsgemäßen Formulierungen wurde durch Zugabe verschiedener carbonylgruppenhaltiger Verbindungen zu handelsüblichen Gebissreinigern nachgewiesen. Als handelübliche Gebissreiniger wurden "Die Blauen" von Kukident® (Reckitt and Colmann AG) und "Blend-a-dent 2 Phasen" (Procter & Gamble) verwendet. Als Bleichtestgewebe wurde Tee auf Baumwolle (WFK, Krefeld) verwendet.

Je vier Lappen Bleichtestgewebe wurden in 200 ml Wasser (38°C) zugeben. Anschließend wurde eine Tablette der handelsüblichen Gebissreiniger sowie 0,1 g einer carbonylgruppenhaltigen Verbindung zugegeben. Reaktionszeit 10 Minuten. Anschließend wurde das Bleichtestgewebe 2x mit Wasser ausgespült und getrocknet. Der Weißgrad des Gewebes wurde mittels eines Elrepho 3000 bestimmt und in Relation zu Weißgrad der unbehandelten Testanschmutzung gesetzt.

### a) Versuche mit "Die Blauen" von Kukident

| | Remissionswert |
|---|---|
| Gebissreiniger ohne Zusatz | + 4.1 |
| Gebissreiniger mit Dipyridylketon-Zusatz | + 5.4 |
| Gebissreiniger mit N-Acetyl-4-piperidon-Zusatz | + 6.3 |
| Gebissreiniger mit Diethylaminoaceton-Zusatz | + 11.0 |

### b) Versuche mit "Blend-a-dent 2 Phasen" (Procter & Gamble)

| | Remissionswert |
|---|---|
| Gebissreiniger ohne Zusatz | + 7.1 |
| Gebissreiniger mit Dipyridylketon-Zusatz | + 8.1 |
| Gebissreiniger mit N-Acetyl-4-piperidon-Zusatz | + 9.4 |
| Gebissreiniger mit Diethylaminoaceton-Zusatz | + 13.2 |

Die Ergebnisse zeigen in beiden Versuchsreihen eine signifikante Zunahme der Bleichleistung, hervorgerufen durch die Zugabe der carbonylgruppenhaltigen Verbindungen.

## Patentansprüche

1. Reinigungsmittel für Zahnprothesen, **dadurch gekennzeichnet, dass** es a) mindestens ein Oxidationsmittel und b) mindestens eine carbonylgruppenhaltige organische Verbindung, die in wässriger Lösung durch Reaktion mit dem Oxidationsmittel eine Dioxiran-Struktur ausbildet, enthält.

2. Reinigungsmittel nach Anspruch 1, **dadurch gekennzeichnet, dass** die carbonylgruppenhaltige Verbindung eine Keton-Struktur aufweist.

3. Reinigungsmittel nach Anspruch 1, **dadurch gekennzeichnet, dass** die carbonylgruppenhaltige organische Verbindung den Formeln 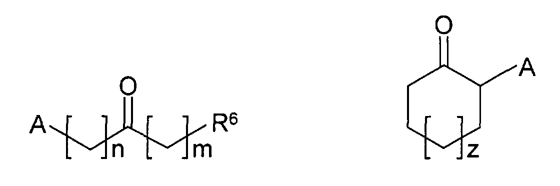 entspricht, wobei A eine Gruppe der Formeln -NR³R⁴ oder -⊕NR³R⁴R⁶ bedeutet, R³, R⁴, R⁵ und R⁶ C₁-C₂₂-Alkyl, C₂-C₂₂-Alkenyl oder Aryl bedeuten, n eine Zahl von 1 bis 11, m eine Zahl von 0 bis 12, z eine Zahl von 0 bis 3 ist und A ein Anion bedeutet.

4. Reinigungsmittel nach Anspruch 1, **dadurch gekennzeichnet, dass** es Alkalimetall- oder Ammoniumperoxosulfate und/oder Alkaliperborate und/oder Alkalipercarbonate enthält.

5. Reinigungsmittel nach Anspruch 1, **dadurch gekennzeichnet, dass** es zusätzlich einen oder mehrere Bleichaktivatoren aus der Gruppe der N-Acyl- oder O-Acyl-Verbindungen enthält.

6. Reinigungsmittel nach Anspruch 1 bis 3, **dadurch gekennzeichnet, dass** es zusätzlich einen oder mehrere Bleichaktivatoren mit einer Nitril-Gruppe enthält.
